(19) Europäisches Patentamt
European Patent Office
Office européen des brevets

(11) Veröffentlichungsnummer: **0 138 137**
**B1**

(12) # EUROPÄISCHE PATENTSCHRIFT

(45) Veröffentlichungstag der Patentschrift: **22.07.87**

(51) Int. Cl.⁴: **C 07 D 491/052**

(21) Anmeldenummer: **84111731.0**

(22) Anmeldetag: **01.10.84**

(54) **4-Thioxo-benzopyrano(2,3-d)pyrimidin-derivate sowie Verfahren zu deren Herstellung.**

(30) Priorität: **30.09.83 DE 3335473**

(43) Veröffentlichungstag der Anmeldung: **24.04.85 Patentblatt 85/17**

(45) Bekanntmachung des Hinweises auf die Patenterteilung: **22.07.87 Patentblatt 87/30**

(84) Benannte Vertragsstaaten: **AT CH DE FR GB IT LI**

(56) Entgegenhaltungen:
**EP-A-0 029 934**
**EP-B-0 008 277**

(73) Patentinhaber: **GÖDECKE AKTIENGESELLSCHAFT, Salzufer 16, D-1000 Berlin 10 (DE)**

(72) Erfinder: **Satzinger, Gerhard, Dr., Im Mattenbühl 7, D-7809 Denzlingen (DE)**
Erfinder: **Barth, Hubert, Dr., Rosenweg 60, D-7830 Emmendingen (DE)**
Erfinder: **Hartenstein, Johannes, Dr., Fohrenbühl 23, D-7801 Stegen- Wittental (DE)**
Erfinder: **Herrmann, Manfred, Dr., Wolfweg 25, D-7811 St. Peter (DE)**
Erfinder: **Fritschi, Edgar, Dr., Am Scheuerwald 2, D-7811 St. Peter (DE)**
Erfinder: **Schütt, Ilse- Dore, Dr., Leimeneckstrasse 8b, D-7804 Glottertal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 138 137 B1

LIBER, STOCKHOLM 1987

## Beschreibung

Gegenstand der vorliegenden Erfindung sind neue 4-Thioxo-benzopyrano [2,3-d] pyrimidin-derivate der allgemeinen Formeln Ia und Ib

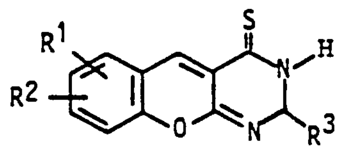

Ia,

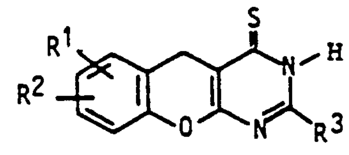

Ib,

im welcher $R^1$ und $R^2$, die gleich oder verschieden sein können, ein Wasserstoff- oder Halogenatom, eine Hydroxylgruppe oder eine geradkettige oder verzweigte Alkoxygruppe mit bis zu 4 Kohlenstoffatomen, oder gemeinsam eine Alkylenoxy- oder eine Alkylendioxygruppe mit 1 bis 3 Kohlenstoffatomen oder einen ankondensierten aromatischen Ring und $R^3$ einen gegebenenfalls durch 1 - 3 Substituenten aus der Gruppe Halogenatome, Dialkylaminogruppen mit bis zu 4 Kohlenstoffatomen oder Alkyl- oder Alkoxygruppen mit bis zu 4 Kohlenstoffatomen sowie bis zu 2 Alkylendioxygruppen mit bis zu 2 Kohlenstoffatomen substituierten Phenylrest bedeuten.

Die Verbindungen Ia und Ib sind Tautomere. Als aromatischer Ring $R^1$ und $R^2$ kommt vor allem der Phenylring in Frage. Bevorzugt ist der unsubstituierte Phenylring in 6,7-Position.

Als Halogenatome kommen Fluor-, Chlor-, Brom- und Jodatome in Frage. Bevorzugt ist das Bromatom.

Als Alkylenoxygruppe kommt vor allem die einen Oxolenring bildende Ethylenoxygruppe in Frage. Als Alkylendioxygruppe ist der Methylendioxyrest bevorzugt, der einen 1,3-Dioxolenring bildet.

Bevorzugt sind Verbindungen der allgemeinen Formeln Ia und Ib, in welchen $R^1$ und $R^2$ gleich oder verschieden sind und ein Wasserstoff- oder Bromatom, eine Hydroxyl-, Methoxy- oder Ethoxygruppe oder zusammen einen unsubstituierten, in 6,7-Position ankondensierten Benzolring und $R^3$ einen unsubstituierten oder durch ein Halogenatom, eine Dimethylamino-, eine Methyl- oder Methylendioxygruppe oder einen durch bis zu 3 Methoxygruppen substituierten Phenylrest darstellt.

Besonders bevorzugt sind Verbindungen der allgemeinen Formeln Ia und Ib, in welchen die Reste $R^1$ und $R^2$ gleich oder verschieden sind und ein Wasserstoff- oder Bromatom oder eine Methoxygruppe und $R^3$ einen Phenyl-, 4-Dimethylaminophenyl-, 4-Methoxyphenyl-, 4-Hydroxyphenyl- oder 4-Methylphenylrest bedeutet.

Ein weiterer Gegenstand der Erfindung ist ein Verfahren zur Herstellung der Verbindungen der allgemeinen Formel Ia und Ib, das dadurch gekennzeichnet ist, daß man in an sich bekannter Weise eine Verbindung der allgemeinen Formel II

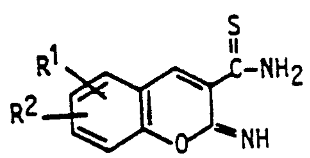

(II),

in welcher $R^1$ und $R^2$ die obengenannte Bedeutung haben,
mit einem aromatischen Aldehyd der allgemeinen Formel III
$R^3 - CHO$ (III),
in welcher $R^3$ die obengenannte Bedeutung hat,
in Gegenwart einer katalytischen Menge einer Base in an sich bekannter Weise, bevorzugt bei Rückflußtemperatur umsetzt und anschließend gewünschtenfalls mit einer starken Base tautomerisiert.

Als Basen haben sich für die katalytische Umsetzung besonders Piperidin und Triethylamin bewährt. Das bei der Reaktion entstehende Wasser entfernt man am einfachsten azeotrop, indem man ein nicht mit Wasser mischbares Lösungsmittel, vie z. B. Methylenchlorid, Chloroform, Benzol oder Toluol als Schlepper verwendet.

Die Reaktionszeit beträgt in der Regel zwischen 6 und 20 Stunden.

Die meist gelb bis orange gefärbten Verbindungen der allgemeinen Formel Ia fallen im Verlauf der Reaktion meist als schwerlösliche Niederschläge aus und können durch Absaugen, Auswaschen und Trocknen isoliert werden, oder aber sie werden nach Entfernen des Lösungsmittels aus dem Rückstand erhalten.

Die Ausgangsprodukte der allgemeinen Formel II erhält man gemäß DE-OS 28 01 353 nach bekannten Vorschriften durch Reaktion von o-Hydroxy-benzaldehyd-Derivaten mit 2-Cyan-thioacetamid.

Die Verbindungen der allgemeinen Formel Ia und Ib sind wertvolle Zwischenprodukte für die Herstellung von 5H-[1]-Benzopyrano-[2,3-d]-Pyrimidin-Derivaten der allgemeinen Formel IV

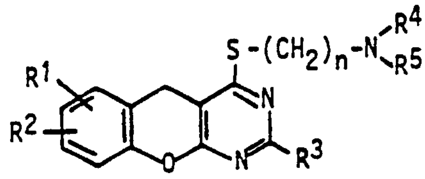 (IV),

in welcher die Reste $R^1$, $R^2$ und $R^3$ die obengenannte Bedeutung haben und $R^4$ und $R^5$, die gleich oder verschieden sein können, ein Wasserstoffatom, eine geradkettige oder verzweigte Alkylgruppe mit bis zu 6 Kohlenstoffatomen oder gemeinsam mit dem Stickstoffatom einen gesättigten 5- oder 6-Ring mit gegebenenfalls weiteren Heteroatomen und n die Zahlen 2 oder 3 bedeuten.

Die Verbindungen der Formel IV sind detailliert in der gleichzeitig hinterlegten Deutschen Offenlegungsschrift Nr. 33 35 472 beschrieben und stellen Arzneimittelwirkstoffe mit einem außergewöhnlichen ulkusprotektiven Wirkungsprofil ohne sekretionshemmende Komponente dar.

Die Herstellung der Verbindungen IV aus den Verbindungen Ia bzw. Ib erfolgt, indem man eine Verbindung der allgemeinen Formel Ia in einem organischen Lösungsmittel mit einer starken Base tautomerisiert und die erhaltene Verbindung der allgemeinen Formel Ib

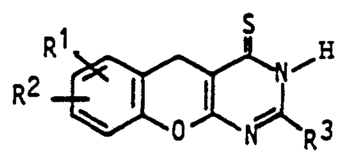

Ib,

in welcher die Reste $R^1$, $R^2$ und $R^3$, die obengenannte Bedeutung haben, gegebenenfalls ohne Isolierung mit einem Alkylierungsmittel der allgemeinen Formel V

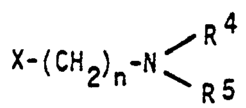 (V),

in welcher die Reste $R^4$ und $R^5$ und n die obengenannte Bedeutung haben und X eine reaktive Ester-gruppe bedeutet,

umsetzt.

Bei dieser Umsetzung werden bevorzugt polare Lösungsmittel, wie z. B. niedere Alkanole verwendet. Bei erhöhter Temperatur beträgt die Reaktionszeit bis zu 6 Stunden. Als Basen kommen z. B. Kaliumcarbonat, Kaliumhydroxid, Natriummethanolat oder Natriumethanolat in Frage.

Die folgenden Beispiele dienen der näheren Erläuterung der Erfindung:

**Beispiel 1**

(Tautomere Form 1a)

2,3-Dihydro-2-phenyl-4-thioxo-[1]-benzopyrano[2,3-d]pyrimidin

10 g 2-Imino-3-thiocarbamoyl-(2H)-chromen, 5,2 g Benzaldehyd und 5 Tropfen Piperidin werden 6 Stunden in 1 l Benzol am Wasserabscheider gekocht. Nach dem Abkühlen wird das ausgefallene gelbe Reaktionsprodukt abgesaugt, mit etwas Benzol ausgewaschen und im Vakuum getrocknet.

Ausbeute 11,0 g, gelbe Kristalle, Fp. 220° C (Zers.).

In analoger Weise erhält man:

2-(4-Chlorphenyl)-2,3-dihydro-4-thioxo-[1]benzopyrano[2,3-d] pyrimidin

Ausbeute 61 %, gelbe Kristalle, Fp 246° C (Zers.)

2-(4-Dimethylaminophenyl)-2,3-dihydro-4-thioxo-[1]benzopyrano[2,3-d]pyrimidin

Ausbeute 63 %, orangefarbene Kristalle, Fp. 218° C (Zers.)

2,3-Dihydro-2-(4-hydroxyphenyl)-4-thioxo-[1]benzopyrano [2,3-d]pyrimidin

Ausbeute 80 %, gelbe Kristalle, Fp 248° C (Zers.)

2,3-Dihydro-2-(4-methylphenyl)-4-thioxo-[1]benzopyrano[2,3-d] pyrimidin
Ausbeute 52 %, gelbe Kristalle, Fp. 205° C (Zers.)
2,3-Dihydro-2-(4-methoxyphenyl)-4-thioxo-[1]benzopyrano [2,3-d] pyrimidin
Ausbeute 52 %, gelbe Kristalle, Fp. 202° C (Zers.)
2,3-Dihydro-2-(3,4-methylendioxyphenyl)-4-thioxo-[1]benzopyrano[2,3-d]pyrimidin
Ausbeute 56 %, gelbe Kristalle, Fp. 226° C (Zers.)
2,3-Dihydro-2-(3,4,5-trimethoxyphenyl)-4-thioxo-[1]benzopyrano[2,3-d]pyrimidin
Ausbeute 85 %, gelbe Kristalle, Fp. 290° C (Zers.)
2,3-Dihydro-7-methoxy-2-phenyl-4-thioxo-[1]benzopyrano[2,3-d] pyrimidin
Ausbeute 87 %, gelbe Kristalle, Fp. 223° C (Zers.)
9,10-Dihydro-9-phenyl-11-thioxo-naphto[1',2':5,6]pyrano [2,3-d]pyrimidin
Ausbeute 85 %, Fp. 320° C (Zers.)
7-Brom-2,3-dihydro-2-phenyl-4-thioxo-[1]benzopyrano[2,3-d] pyrimidin
Ausbeute 80 %, Fp. 330° C (Zers.)
9-Ethoxy-2,3-dihydro-2-phenyl-4-thioxo-[1]benzopyrano[2,3-d] pyrimidin
Ausbeute 77 %, Fp. 215° C (Zers.)
2,3-Dihydro-6,8-dimethoxy-2-phenyl-4-thioxo-[1]benzopyrano [2,3-d]pyrimidin
Ausbeute 68 %, Fp. 298° C (Zers.)
2,3-Dihydro-7-Methoxy-2-(4-methoxyphenyl)-4-thioxo-[1]benzopyrano[2,3-d]pyrimidin
Ausbeute 72 %, Fp. 250° C (Zers.)
7-Brom-2,3-dihydro-2-(4-methoxyphenyl)-4-thioxo-[1]benzopyrano[2,3-d]pyrimidin
Ausbeute 75 %, Fp. 235° C (Zers.)
2,3-Dihydro-7,8-methylendioxy-2-(3,4-methylendioxyphenyl)-4-thioxo-[1]benzopyrano[2,3-d]pyrimidin
Ausbeute 93 %, gelbe Kristalle, Fp. 270° C (Zers.)
2,3,7,8-Tetrahydro-8-(4-methylphenyl)-6-thioxo-furo[2',3': 7,8][1]benzopyrano[2,3-d]pyrimidin
Ausbeute 80 %, gelbe Kristalle, Fp. ab 210° C (Zers.)

**Beispiel 2**

(Tautomere Form 1b)
2-Phenyl-4-thioxo-3H,5H-[1]benzopyrano[2,3-d]pyrimidin

Man löst 1,1 g Natrium in 300 ml abs. Ethanol, gibt 14,6 g 2,3-Dihydro-2-phenyl-4-thioxo-[1]-benzopyrano[2,3-d]pyrimidin hinzu und erhitzt 1 Stunde zum Sieden. Danach engt man die Reaktionsmischung am Rotationsverdampfer ein, gießt in 300 ml Wasser und säuert vorsichtig mit verdünnter Salzsäure an. Der ausgefallene Niederschlag wird abgesaugt und aus Dimethylformamid/Ethanol umkristallisiert.
Ausbeute 80 % gelbe Kristalle, Fp. 245° C (Zers.)
In analoger Weise werden erhalten:
2-(4-Dimethylaminophenyl)-4-thioxo-3H,5H-[1]benzopyrano [2,3-d]pyrimidin
Ausbeute 76 %, gelbe Kristalle, Fp. 245° C (Zers.)
2-(4-Methoxyphenyl)-4-thioxo-3H,5H-[1]benzopyrano[2,3-d] pyrimidin
Ausbeute 74 %, gelbe Kristalle, Fp. 265° C (Zers.)
2-(4-Chlorphenyl)-4-thioxo-3H,5H-[1]benzopyrano[2,3-d] pyrimidin
Ausbeute 70 %, gelbe Kristalle, Fp. 340° C (Zers.)
4-Thioxo-2-(3,4,5-trimethoxyphenyl)-3H,5H-[1]benzopyrano [2,3-d]pyrimidin
Ausbeute 79 %, ockerfarbene Kristalle, Fp. 290° C (Zers.)
2-(4-Hydroxyphenyl)-4-thioxo-3H,5H-[1]benzopyrano[2,3-d] pyrimidin
Ausbeute 65 %, gelbliche Kristalle, Fp. 274° C, (Zers.)

**Patentansprüche**

für die Vertragsstaaten CH, DE, FR, GB, IT, LI.

1. 4-Thioxo-benzopyrano[2,3-d]-pyrimidin-derivate der allgemeinen Formel Ia und Ib

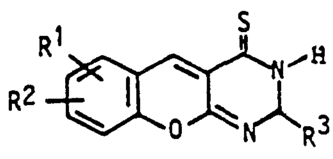

Ia,

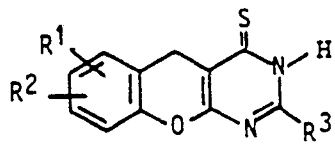

Ib,

in welcher $R^1$ und $R^2$, die gleich oder verschieden sein können, ein Wasserstoff- oder Halogenatom, eine Hydroxylgruppe oder eine geradkettige oder verzweigte Alkoxygruppe mit bis zu 4 Kohlenstoffatomen, oder gemeinsam eine Alkylenoxy- oder eine Alkylendioxygruppe mit 1 bis 3 Kohlenstoffatomen oder einen ankondensierten aromatischen Ring und $R^3$ einen gegebenenfalls durch 1-3 Substituenten aus der Gruppe Halogenatome, Dialkylaminogruppen mit bis zu 4 Kohlenstoffatomen oder Alkyl- oder Alkoxygruppen mit bis zu 4 Kohlenstoffatomen sowie bis zu 2 Alkylendioxygruppen mit bis zu 2 Kohlenstoffatomen substituierten Phenylrest bedeuten.

2. Verfahren zur Herstellung von 4-Thioxo-benzopyrano[2,3-d] pyrimidin-derivaten der allgemeinen Formeln Ia und Ib, dadurch gekennzeichnet, daß man in an sich bekannter Weise eine Verbindung der allgemeinen Formel II

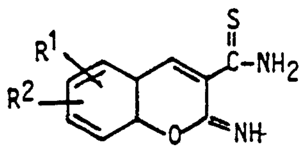

(II),

in welcher $R^1$ und $R^2$ die obengenannte Bedeutung haben,
mit einem aromatischen Aldehyd der allgemeinen Formel III
$R^3 - CHO$ (III),
in welcher $R^3$ die obengenannte Bedeutung hat,
in Gegenwart einer katalytischen Menge einer Base umsetzt und die erhaltene Verbindung Ia anschließend gewünschtenfalls mit einer starken Base tautomerisiert.

**Patentansprüche**

für den Vertragsstaat AT

1. Verfahren zur Herstellung von 4-Thioxo-benzopyrano[2,3-d] pyrimidin-derivaten der allgemeinen Formeln Ia und Ib,

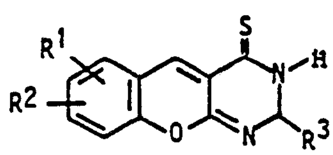

Ia,

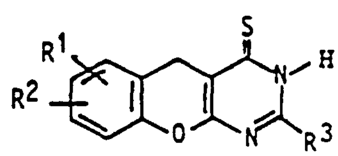

Ib,

in welcher $R^1$ und $R^2$, die gleich oder verschieden sein können, ein Wasserstoff- oder Halogenatom, eine Hydroxylgruppe oder eine geradkettige oder verzweigte Alkoxygruppe mit bis zu 4 Kohlenstoffatomen, oder gemeinsam eine Alkylenoxy- oder eine Alkylendioxygruppe mit 1 bis 3 Kohlenstoffatomen oder einen ankondensierten aromatischen Ring und $R^3$ einen gegebenenfalls durch 1-3 Substituenten aus der Gruppe Halogenatome, Dialkylaminogruppen mit bis zu 4 Kohlenstoffatomen oder Alkyl- oder Alkoxygruppen mit bis zu 4 Kohlenstoffatomen sowie bis zu 2 Alkylendioxygruppen mit bis zu 2 Kohlenstoffatomen substituierten Phenylrest bedeuten,
dadurch gekennzeichnet, daß man in an sich bekannter Weise eine Verbindung der allgemeinen Formel II

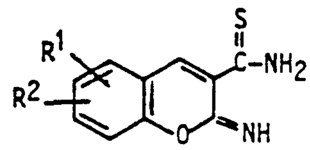

(II),

in welcher $R^1$ und $R^2$ die obengenannte Bedeutung haben,
mit einem aromatischen Aldehyd der allgemeinen Formel III
$R^3 - CHO$ (III),
in welcher $R^3$ die obengenannte Bedeutung hat,
in Gegenwart einer katalytischen Menge einer Base umsetzt und die erhaltene Verbindung Ia anschließend gewünschtenfalls mit einer starken Base tautomerisiert.

**Claims**

for the contracting states CH, DE, FR, GB, IT, LI

1. 4-thioxo-benzopyrano[2,3-d]-pyrimidine derivatives of general formulae Ia and Ib,

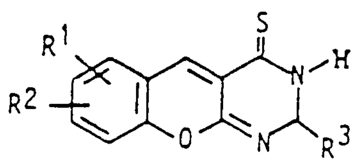

Ia

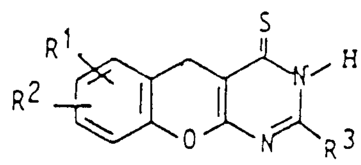

Ib

in which $R^1$ and $R^2$, which may be the same or different, represent a hydrogen or halogen atom, a hydroxyl group or a straight-chain or branched alkoxy group with up to four carbon atoms or together represent an alkylenoxy or alkylendioxy group with 1 to 3 carbon atoms or a condensed aromatic ring and $R^3$ a phenyl radical with may be substituted by 1 to 3 substituents, which may be halogen atoms, dialkylamino groups with up to 4 carbons, alkyl- or alkoxy groups with up to 4 carbons, or up to 2 alkylene dioxy groups with up to 2 carbons.

2. Process for the preparation of 4-thioxo-benzopyrano [2,3-d] pyrimidine derivatives of the general formulae Ia and Ib, characterized by that in a known manner a compound of the general formula II,

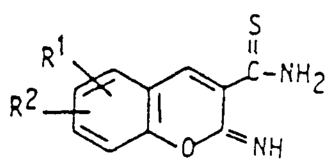

(II)

wherein $R^1$ and $R^2$ have the above meaning, is reacted with an aromatic aldehyde of the general formula III, $R^3 - CHO$ (III), in which $R^3$ has the above meanings, in the presence of a catalytic amount of a base and compound Ia thus obtained subsequently is tautomerized with a strong acid if desired.

**Claim**

for the contracting state: AT

1. Process for the preparation of 4-thioxo-benzopyrano [2,3-d] pyrimidine derivatives of general formulae Ia and Ib,

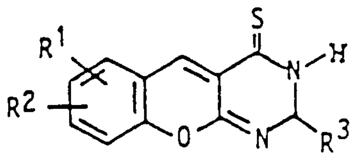 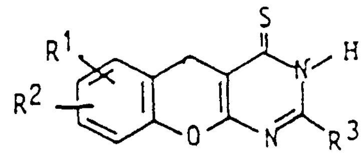

Ia Ib

in which $R^1$ and $R^2$, which may be the same or different, represent a hydrogen or halogen atom, a hydroxyl group or a straight-chain or branched alkoxy group with up to 4 carbon atoms or together represent an alkylenoxy or alkylendioxy group with up to 3 carbon atoms or a condensed aromatic ring, and $R^3$ a phenyl radical which may be substituted by 1 to 3 substituents, which may be halogen atoms, dialkylamino groups with up to 4 carbons, alkyl or alkoxy groups with up to 4 carbon atoms or up to 2 alkylene dioxy groups with up to 2 carbons characterized by that in a known manner a compound of the general formula II,

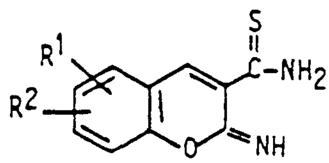 (II)

in which $R^1$ and $R^2$ have the above meanings, is reacted with an aromatic aldehyde or the general formula III,
$R^3$ — CHO (III)
in which $R^3$ has the above meanings, in the presence of a catalytic amount of a base and the compound Ia thus obtained subsequently is tautomerized with a strong acid if desired.

**Revendications**

pour les Etats contractants: CH, DE, FR, GB, IT, LI.

1. Dérivés de 4-thioxo-benzopyrano(2,3-d)pyrimidine de formules générales Ia et Ib:

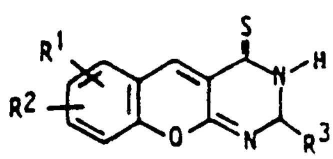 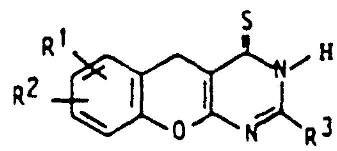

Ia, Ib,

dans lesquelles:

$R^1$ et $R^2$, qui peuvent être identiques ou différents, représentent un atome d'hydrogène ou un atome d'halogène, un groupe hydroxyle ou un groupe alkoxy à chaîne droite ou ramifiée renfermant jusqu'à 4 atomes de carbone, ou forment ensemble un groupe alkylèneoxy ou alkylènedioxy renfermant de 1 à 3 atomes de carbone, ou un cycle aromatique condensé sur le nuyau; et

$R^3$ représente un radical phenyle, éventuellement substitué par 1 à 3 substituants choisis dans le groupe constitué par un atome d'halogène, des groupes dialkylamino renfermant jusqu'à 4 atomes de carbone ou des groupes alkyle ou alkoxy renfermant jusqu'à 4 atomes de carbone, ainsi que jusqu'à deux groupes alkylènedioxy renfermant jusqu'à 2 atomes de carbone.

2. Procédé de préparation de dérivés de 4-thioxo-benzopyrano(2,3-d)-pyrimidine de formules générales Ia et Ib, caractérisé en ce que l'on fait réagir un composé de formule générale II:

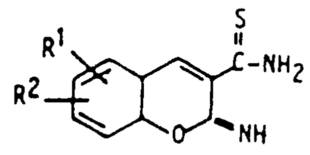

(II).

dans laquelle $R^1$ et $R^2$ ont la signification indiquée ci-dessus, avec un aldéhyde aromatique de formule générale III

$R^3 - CHO$ (III)

dans laquelle $R^3$ a la signification ci-dessus,

en présence d'une quantité catalytique d'une base et, le cas échéant, on tautomérise le composé la obtenu à l'aide d'une base forte.

**Revendication**

pour l'Etat contractant: AT

1. Procédé de préparation de dérivés de 4-thioxo-benzopyrano(2,3-d)-pyrimidine de formules générales Ia et Ib:

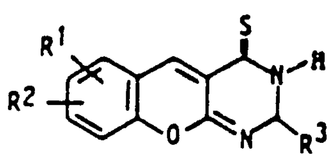

Ia,

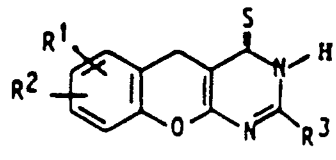

Ib,

dans lesquelles:

$R^1$ et $R^2$, qui peuvent être identiques ou différents, représentent un atome d'hydrogène ou un atome d'halogène, un groupe hydroxyle ou un groupe alkoxy à chaîne droite ou ramifiée renfermant jusqu'à 4 atomes de carbone, ou forment ensemble un groupe alkylèneoxy ou alkylènedioxy renfermant de 1 à 3 atomes de carbone, ou un cycle aromatique condensé sur le noyau; et

$R^3$ représente un radical phényle, éventuellement substitué par 1 à 3 substituants choisis dans le groupe constitué par un atome d'halogène, des groupes dialkylamino renfermant jusqu'à 4 atomes de carbone ou des groupes alkyle ou alkoxy renfermant jusqu'à 4 atomes de carbone, ainsi que jusqu'à deux groupes alkylènedioxy renfermant jusqu'à 2 atomes de carbone.

caractérisé en ce que l'on fait réagir, d'une façon connue en soi, un composé de formule générale II:

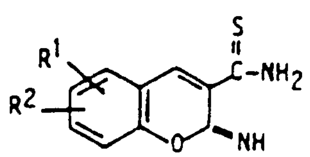

(II).

dans laquelle $R^1$ et $R^2$ ont la signification indiquée ci-dessus, avec un aldéhyde aromatique de formule générale III:

$R^3 - CHO$ (III)

dans laquelle $R^3$ a la signification ci-dessus, en présence d'une quantité catalytique d'une base et l'on tautomérise, le cas échéant, le composé la obtenu à l'aide d'une base forte.